# EUROPÄISCHE PATENTSCHRIFT

(11) **EP 1 528 752 B1**
(45) Veröffentlichungstag und Bekanntmachung des Hinweises auf die Patenterteilung: **05.12.2007**
(21) Anmeldenummer: 04023327.2
(22) Anmeldetag: 30.09.2004
(51) Int. Cl.: H04L 29/08

(54) **Kommunikationssystem und Verfahren zur Bearbeitung medizinischer Daten**
A communication system and method for handling of medical data
Un système et une méthode de communication pour la manipulation des données médicales

(30) Priorität: 29.10.2003 DE 10350538
(43) Veröffentlichungstag der Anmeldung: 04.05.2005
(73) Patentinhaber: Corscience GmbH & Co. KG, 91052 Erlangen (DE)
(72) Erfinder: Gmelin, Moritz, Dipl.-Inf., 76137 Karlsruhe (DE); Bolz, Armin, Prof. Dr., 91054 Buckenhof (DE)
(74) Vertreter: Herrmann, Uwe

(56) Entgegenhaltungen:
- US-A1- 2002 049 684
- US-B1- 6 272 545
- SESHADRI P ET AL: "SQLServer for Windows CE-a database engine for mobile and embedded platforms" DATA ENGINEERING, 2000. PROCEEDINGS. 16TH INTERNATIONAL CONFERENCE ON SAN DIEGO, CA, USA 29 FEB.-3 MARCH 2000, LOS ALAMITOS, CA, USA,IEEE COMPUT. SOC, US, 29. Februar 2000 (2000-02-29), Seiten 642-644, XP010378761 ISBN: 0-7695-0506-6

## Beschreibung

Die vorliegende Erfindung betrifft ein Kommunikationssystem sowie ein Verfahren zur Bearbeitung medizinischer Daten gemäß dem Oberbegriff der Ansprüche 1 und 24. Insbesondere bei der Erstversorgung und Nachbehandlung von Notfallpatienten ist es von großer Bedeutung, dass die im Rahmen der Behandlung angefallenen Daten zuverlässig erfasst und von dem behandelnden Arzt zeitgleich oder zeitnah eingesehen und bei Bedarf vervollständigt werden können. Um die von medizinischen Geräten erfassten Daten einem größeren Kreis von Anwendern zur Einsicht zur Verfügung zu stellen, ist vorgeschlagen worden, die Daten auf einem Web-Server abzulegen. Ein derartiges System ist aus der WO 02/17593 A2 bekannt. Das darin offenbarte System ermöglicht die Visualisierung von Behandlungsdaten, die beispielsweise von einem Herzschrittmacher drahtlos an eine Kommunikationseinrichtung und von dieser an einen Web-Server übertragen werden. Dadurch ist die Beobachtung des Patienten ohne die Notwendigkeit eines Arztbesuches möglich. Die in dem Web-Server abgelegten Daten können mittels eines Internet-Browsers von dazu berechtigen Anwendern eingesehen werden.

Das aus der WO 02/17593 A2 bekannte System weist den Nachteil auf, dass sich die Behandlungsdaten und Anwenderprogramme auf dem zentralen Web-Server befinden, von dem aus die benötigten Dateien im Bedarfsfall auf einen lokalen Rechner geladen werden müssen und hier mittels des Browsers visualisiert werden können. Bei einer derartigen Browser-Anwendung werden bei jedem Aufruf sämtliche Daten bzw. Applikationen von dem zentralen Web-Server auf den lokalen Rechner geladen, wodurch sich verhältnismäßig große Mengen zu übertragender Daten und entsprechend lange Übertragungszeiten ergeben. Ein weiterer Nachteil ergibt sich daraus, dass Anwender ohne Internetanschluss bei einem derartigen System naturgemäß nicht die Möglichkeit der Einsicht in die auf dem Web-Server befindlichen Behandlungsdaten haben. Bei dem aus der WO 02/17593 A2 bekannten System ist schließlich von Nachteil, dass aufgrund der Übertragung vollständiger Datensätze grundsätzlich auch die Möglichkeit besteht, diese unbefugt abzuhören bzw. abzurufen, was bei den sensiblen Patienten- und Behandlungsdaten selbstverständlich unerwünscht ist.

Aus dem Artikel "SQL Server for Windows CE - A data base engine for mobile and embedded platforms; Seshadri, P. et. al, Data Engineering, 2000, Proceedings, 16th International Conference on San Diego, CA, USA, 29 FEB - 3 MAR 2000, Los Alamitos, CA, USA, IEEE Compute. SOC, US, 29. Feb. 2000 (2000-02-29)", Seiten 642 bis 644, XP 010378761 ist eine Datenbank für mobile Plattformen bekannt, bei der der Datenaustausch zwischen Server und Client derart vorgenommen wird, dass Änderungen, die im Client vorgenommen werden, zum Zwecke der Synchronisation an den Server übertragen werden.

Die US 2002/0049684 A1 betrifft ein Client-Server-Kommunikationssystem, bei dem Änderungen, die in einem Client vorgenommen werden, auf einem Server gespeichert werden. Diese Daten werden sodann von dem Server an ein medizinisches Gerät übermittelt, um dort aktuelle medizinische Daten bzw. einen Behandlungsplan bereitzustellen.

Der Erfindung liegt daher die Aufgabe zugrunde, ein Kommunikationssystem sowie ein Verfahren zur Bearbeitung medizinischer Daten bereitzustellen, das mit geringen Daten-Übertragungszeiten arbeitet, das auch für Anwender ohne Internetzugang zugänglich ist und bei dem mit geringem Umfang der zu übertragenden Datenmenge die Erzeugung identischer Datensätze in den Clients und im Server möglich ist.

Diese Aufgabe wird durch ein Kommunikationssystem mit den Merkmalen des Patentanspruchs 1 sowie durch ein Verfahren mit den Merkmalen des Patentanspruchs 24 gelöst. Vorteilhafte Ausgestaltungen der Erfindung sind Gegenstand der Unteransprüche. Das vorliegende Kommunikationssystem betrifft eine Client-Server-Architektur, bei der auf dem Client eine lokal abgespeicherte Auswerteapplikation lokal aufgerufen werden kann. Mittels der Auswerteapplikation ist es möglich, lokal gespeicherte Daten zu visualisieren und zu verarbeiten. Die Arbeit mit lokal gespeicherten Daten verringert das Risiko, dass sensible Daten unbefugt abgehört, abgerufen oder eingesehen werden können. Die an dem Client aufbereiteten, geänderten oder ergänzten Daten sind an den Server übermittelbar und dort speicherbar und stehen sodann für weitere Anwender zur Verfügung. Der Client erhält die medizinischen Daten beispielsweise entweder unmittelbar von dem medizinischen Gerät oder vom Server, sofern diese bereits im Server abrufbar vorliegen.

Unter dem Begriff medizinische Daten sind sämtliche im Rahmen einer Patientenversorgung oder -behandlung relevanten Daten zu verstehen, wie z.B. Behandlungsdaten, die während einer ärztlichen Behandlung anfallen, oder auch ohne Arzteinwirkung anfallende Daten, wie beispielsweise die kontinuierlich aufgezeichneten Daten eines Herzschrittmachers, Blutzuckersensors etc.

Das Vorhandensein einer lokalen Applikation auf dem Client bringt den Vorteil mit sich, dass auch Anwender ohne Internetanschluss bedient werden können und dass das Laden einer Applikation von einem zentralen Server entfällt, wodurch sich die zu übertragende Datenmenge reduzieren lässt. Bei einem Datenaustausch zwischen Server und Client zum Zwecke der Synchronisation kann in vorteilhafter Ausgestaltung der Erfindung weiter vorgesehen sein, dass nicht der gesamte Datensatz übertragen wird, sondern nur die Differenzmenge in Form neuer oder geänderter Daten, wodurch sich die Übertragungszeiten weiter verringern lassen. Aufgrund der Möglichkeit, Daten von dem oder den Clients auf den Server zu übertragen, kann sichergestellt werden, dass der auf dem Server befindliche Datensatz stets aktualisiert ist.

Die Datenübertragung zwischen den medizinischen Gerät, dem oder den Clients und dem Server kann jeweils unmittelbar oder mittelbar durch Zwischenschaltung weiterer Komponenten des Systems erfolgen.

Die in dem medizinischen Gerät gespeicherten Daten können über dessen Kommunikationsschnittstelle unmittelbar an den oder die Clients oder an den Server übermittelt werden.

Bei dem medizinischen Gerät kann es sich beispielsweise um einen externen Defibrillator handeln. Grundsätzlich eignet sich das erfindungsgemäße Kommunikationssystem und Verfahren auch für beliebige andere medizinische Geräte, vorzugsweise für transportable medizinische Geräte, die beispielsweise bei Notfalleinsätzen Verwendung finden.

Wie oben ausgeführt, sind erfindungsgemäß Mittel zur Synchronisation der Daten des Servers und des Clients vorgesehen, mittels derer ausschließlich in dem Client aufbereitete, geänderte oder ergänzte Daten an den Server übertragbar sind. Somit werden bei einer Kommunikation zwischen Server und Client nicht stets sämtliche Daten eines Datenobjektes neu übertragen, sondern nur die Differenzmenge. Entsprechendes gilt für die von weiteren Clients vorgenommenen Änderungen, die auf dem Server abgelegt sind. Auch für derartige Änderungen gilt, dass mittels der Synchronisationseinheit nur die von den weiteren Clients vorgenommenen Änderungen oder Ergänzungen von dem Server auf den synchronisierenden Client übertragen werden. Damit lässt sich sicherstellen, dass in sämtlichen Clients sowie im Server identische Datensätze vorliegen, wobei verhältnismäßig geringe Datenmengen zu übertragen sind.

In weiterer Ausgestaltung der Erfindung ist vorgesehen, dass es sich bei der auf dem Client befindlichen Auswerteapplikation um eine Java WebStart Anwendung handelt. Ein Vorteil dieser Technologie ist es, dass der Anwender Updates der Software automatisch über das Internet erhalten kann. Dabei können die neuesten Komponenten der Software beim Start installiert werden, sofern eine Internet-Verbindung besteht. Ein weiterer Vorteil der Java WebStart Anwendung ergibt sich daraus, dass diese plattformunabhängig ist und dementsprechend unabhängig vom Betriebssystem des Anwenders eingesetzt werden kann.

In weiterer Ausgestaltung der Erfindung ist vorgesehen, dass wenigstens eine Kommunikationsschnittstelle des medizinischen Geräts sowie wenigstens eines Clients eine Schnittstelle zur Nahübertragung von Daten ist. Die Wahl der Datenübertragungstechnik ist beliebig. Beispielsweise kommen Infrarot oder Bluetooth in Frage. Ferner kann vorgesehen sein, dass eine Technik Anwendung findet, die üblicherweise nur zur Datenfernübertragung dient, wie beispielsweise die Mobilfunktechnik. Hinsichtlich der Technik der Datenübertragung zwischen medizinischem Gerät und Client gibt es keinerlei Beschränkungen. Zur Übergabe der Daten des medizinischen Geräts kann dieses über eine Infrarotschnittstelle oder auch beispielsweise per Bluetooth mit dem Client kommunizieren. Die Daten können sodann nach einer eventuellen Aufbereitung im Client von diesem auf den Server übertragen werden. Die Schnittstelle des medizinischen Geräts kann des weiteren dazu dienen, von dem Client Daten zu übernehmen, wie beispielsweise Standort-Konfigurationsdaten oder Geräte-Einstellungsdaten. Grundsätzlich ist es ebenso möglich, derartige Daten unmittelbar vom Server an das medizinische Gerät zu senden.

In bevorzugter Ausgestaltung der Erfindung ist eine Kommunikationsschnittstelle des medizinischen Geräts als Mobilfunkschnittstelle ausgeführt. Der Server oder ein Kommunikationsserver verfügen bei dieser Ausgestaltung der Erfindung ebenfalls über eine Mobilfunkschnittstelle, mittels derer Daten von dem medizinischen Gerät an den Server oder an den Kommunikationsserver und von diesem an den Server übertragbar sind. Auf diese Weise können Einsatz- bzw. Behandlungsdaten direkt vom medizinischen Gerät auf den Server übertragen werden. Die Übertragung kann mittels Mobilfunktechnik oder auch anderer geeigneter Datenübertragungstechniken erfolgen. Wird die Mobilfunktechnik verwendet, kommt beispielsweise der GSM-Standard, GPRS oder UMTS zum Einsatz.

Der Kommunikationsserver kann Bestandteil des Servers sein.

Weist das medizinische Gerät sowie der Server oder ein Kommunikationsserver eine Mobilfunkschnittstelle auf, kann vorgesehen sein, dass Daten von dem Server an das medizinische Gerät oder von dem Server an den Kommunikationsserver und von diesem an das medizinische Gerät übertragbar sind. Auf diese Weise ist es möglich, das medizinische Gerät schnell mit wichtigen Daten, beispielsweise mit Patientendaten, die zur weiteren Behandlung erforderlich oder nützlich sind, zu versorgen. Außerdem ist es möglich, das medizinische Gerät über eine derartige Datenverbindung mit Standortdaten oder Konfigurationsdaten zu versehen, ohne dass dieses dazu mit einem Client kommunizieren müsste.

In der Speichereinheit des Servers oder des Clients können unter anderem für den Standort des medizinischen Gerätes spezifische Daten, Firmware-Updates, Daten betreffend Geräteeinstellungen des medizinischen Gerätes oder Patientendaten, insbesondere Vorerkrankungen und aktuelle oder vergangene Behandlungen betreffende Daten abgelegt sein. Diese Daten oder Updates können mittels Kommunikationsschnittstellen des Servers, des Clients und des medizinischen Gerätes unmittelbar vom Server oder vom Client an das medizinsche Gerät oder vom Server an den Client und mittels der Kommunikationsschnittstellen des Clients und des medizinischen Gerätes vom Client zum medizinischen Gerät übertragbar sein. Auf diese Weise ist das medizinische Gerät mittels des Clients und/oder mittels des Servers konfigurierbar und kann mit relevanten Daten, beispielsweise wichtigen und sofort benötigten Patientendaten versorgt werden.

In weiterer Ausgestaltung der Erfindung ist vorgesehen, dass das medizinische Gerät über Mittel zur Erfassung von im Rahmen einer Behandlung angefallenen Messwerten verfügt. Die Mittel zur Messwerterfassung können Detektoren umfassen, mittels derer von Sensoren per Nahübertragungstechnik, z.B. per Infrarot oder Bluetooth übertragene Daten erfassbar sind. Besonders vorteilhaft ist es, wenn das medizinische Gerät selbständig Sensoren in Reichweite erkennt, die relevante Daten senden. Die Daten dieser Sensoren werden beispielsweise zusätzlich zu dem EKG im Speicher des medizinischen Gerätes bzw. eines Defibrillators abgelegt und stehen beim Auslesen der Daten zur Verfügung. Handelt es sich bei dem medizinischen Gerät um einen Defibrillator, können die Mittel zur Messwerterfassung die Elektroden des Defibrillators umfassen. Die Datenverarbeitung auf dem Defibrillator kann sich zum Einen auf die Visualisierung von Daten, insbesondere auf die Darstellung des aufgezeichneten EKGs in Echtzeit beziehen. Parallel dazu kann auf dem Monitor noch in numerischer Form die Pulsfrequenz und eventuell die Sauerstoffsättigung angezeigt werden. Hierzu kann ein externer spO₂-Sensor per Bluetooth in die Datenerfassung eingebunden werden. Die Datenverarbeitung des Defibrillators kann ferner die Protokollierung hinsichtlich der Teile des Protokolls umfassen, die zeitnah zu erfassen sind. Die Datenerfassung kann später mit Hilfe des Clients 30 vervollständigt werden.

Weiterhin kann vorgesehen sein, dass das medizinische Gerät Mittel zur Erfassung von Patientendaten aufweist. Dabei kann es sich beispielsweise um Lesemittel für beliebige Speichermedien, auf denen Patientendaten abgelegt sind, handeln. Eine Möglichkeit ist ein Chipkarten-Lesegerät zum Auslesen einer Versichertenkarte des Patienten. Auch ist es denkbar, dass die Mittel Ein- und/oder Ausgabemittel aufweisen, mittels derer Daten bzw. ein Code eingegeben werden, die eine Zuordnung zu einem Patienten aufweisen und das Einlesen der entsprechenden Patientendaten in das medizinische Gerät und deren Ausgabe ermöglichen. Im Anschluß an die Eingabe der Daten bzw. des Codes kann vorgesehen sein, dass das medizinische Gerät aus einem beliebigen Speicher auf die Patientendaten zugreift. Es kann vorgesehen sein, dass die Mittel zum Herunterladen der Patientendaten aus einer elektronischen Patientenakte per Internet dienen. In diesem Fall sind die Mittel als Vorrichtungen zum Download der entsprechenden Daten aus dem Internet oder auch von einem beliebigen anderen, nicht web-basierten Server ausgeführt. Durch die Mittel zur Erfassung von Patientendaten kann sichergestellt werden, dass Basisinformationen zum Patienten im medizinischen Gerät vorliegen. Alle darüber hinausgehenden Informationen können in dem medizinischen Gerät oder mittels des Clients eingegeben werden.

Das medizinische Gerät weist in weiterer Ausgestaltung der Erfindung Mittel zur Eingabe und/oder zur Auswahl von Daten auf. Damit wird die Möglichkeit eröffnet, bereits während des Einsatzes oder der Behandlung Daten einzugeben, die später zu einem Einsatzprotokoll vervollständigt werden können. Liegen an dem medizinischen Gerät nur begrenzte Eingabemöglichkeiten vor, ist es vorteilhaft, wenn nur eine Auswahl vorgegebener Daten erfolgt. Die entsprechenden Listen, die die vorgegebenen Auswahlantworten enthalten, können in der Standortakte des Servers abgelegt sein und von dort oder über den Client in das medizinische Gerät eingelesen werden.

Ist das medizinische Gerät während der Behandlung schwer einsehbar, ist es von Vorteil, wenn dieses über eine Kommunikationsschnittstelle verfügt, mittels derer im Rahmen einer Behandlung angefallene Messwerte vorzugsweise in Echtzeit an einen Monitor übertragbar sind. Dabei ist beispielsweise an die Darstellung eines EKGs zu denken. Die Übertragung der Messwerte kann beispielsweise per Bluetooth erfolgen. Grundsätzlich sind auch andere Übertragungstechniken denkbar. Alternativ oder zusätzlich kann das medizinische Gerät einen integrierten Monitor zur Visualisierung erfasster Behandlungsdaten aufweisen, was insbesondere dann vorteilhaft ist, wenn ein externer Monitor bzw. eine Übertragungsmöglichkeit zu einem externen Monitor nicht vorhanden ist.

In weiterer Ausgestaltung der Erfindung ist vorgesehen, dass das Kommunikationssystem eine Empfangseinheit mit einer Kommunikationsschnittstelle zum Empfang der in dem medizinischen Gerät abrufbar gespeicherten Daten beispielsweise mittels Infrarot oder Bluetooth aufweist. Die Empfangseinheit kann sich beispielsweise in der Notaufnahme einer Klinik befinden und dient dazu, die in dem medizinischen Gerät befindlichen Behandlungsdaten rasch abzurufen. Die auf diese Weise übertragenen Daten können dann beispielsweise weiterverarbeitet werden und/oder in das Intranet einer Klinik eingestellt werden. Des weiteren kann vorgesehen sein, dass die Empfangseinheit über eine Schnittstelle zum Datenaustausch mit dem Server verfügt, so dass die Behandlungsdaten oder ergänzende Daten von dem Server abgerufen werden können, was allerdings längere Übertragungszeiten zur Folge hat.

In weiterer Ausgestaltung der Erfindung ist vorgesehen, dass der Server über mehrere Datensätze verfügt, von denen wenigstens ein erster Datensatz Patientendaten und wenigstens ein zweiter Datensatz das medizinische Gerät betreffende Daten umfasst, wobei die in dem zweiten Datensatz abgelegten Daten keine Zuordnung zu einem bestimmten Patienten aufweisen. In dem zweiten Datensatz werden ausschließlich Daten eines betreffenden Gerätes abgelegt. Diese Daten weisen aus datenschutzrechtlichen Gründen keinen Bezug zu einem bestimmten Patienten auf.

Des weiteren kann vorgesehen sein, dass der Server wenigstens einen dritten Datensatz mit für einen Arzt spezifischen Daten und wenigstens einen vierten Datensatz mit für den Standort des medizinischen Gerätes spezifischen Daten umfasst. Bei den arztspezifischen Daten kann es sich beispielsweise um persönliche Daten des Arztes, Daten seiner Einsätze, persönliche Einstellungen des medizinischen Gerätes etc. handeln. Bei den standortspezifischen Daten des vierten Datensatzes kann es sich beispielsweise um Daten bezüglich des möglichen Bedienpersonals am Standort, um eine Liste der medizinischen Geräte des Standortes, Kliniken, Fahrzeuge oder Medikamente des Standortes sowie um Einsätze handeln, die mit den medizinischen Geräten des Standortes erbracht wurden.

Besonders vorteilhaft ist es, wenn der Server über eine Kommunikationsschnittstelle verfügt, mittels derer Daten von per Telefax übermittelten Dokumenten in den Server einlesbar sind. Beispielsweise kann jede Datei des Servers mit einer Fax-ID versehen werden, die eingehende Telefaxe auf dem Server identifiziert und diese dann als neues Dokument in der entsprechenden Datei archiviert. Des weiteren ist es möglich, dass der Server über eine Kommunikationsschnittstelle verfügt, mittels derer auf dem Server gespeicherte Daten per Telefax übermittelbar sind. Dabei ist es denkbar, dass der Versender den Server anweist, ein Fax mit relevanten Daten an einen definierten Empfänger zu senden. Denkbar ist es ebenfalls, automatisch Daten, beispielsweise Einsatzprotokolle an alle Kliniken eines Standortes zu versehen. Ebenfalls ist es möglich, dass Fax-Abruf-Kennungen zugeordnet werden, die ein direktes Abrufen von Daten, beispielsweise von Daten des letzten Einsatzes per Fax-Polling vom Server erlauben.

In einer weiteren Ausgestaltung der Erfindung sind in der Speichereinheit des Servers mit den darin abgelegten Daten verknüpfte Bedingungen oder Grenzwerte abgelegt, wobei Mittel vorgesehen sind, mittels derer bei Eintreten der Bedingungen oder bei Erreichen des Grenzwertes eine Meldung oder Mitteilung generierbar ist. Beispielsweise ist es denkbar, dass bei Überschreitung eines Grenzwertes eines Blutzuckerwertes ein Arzt per SMS oder Email benachrichtigt wird. Auch ist es denkbar, dass Nachrichten über den Zustand des medizinischen Gerätes in dem Server abgelegt werden, der den Versand eines Telefaxes an den zuständigen Service-Beauftragten veranlasst.

Die Erfindung betrifft ferner ein Verfahren gemäß Anspruch 24 zur Bearbeitung medizinischer Daten, bei dem in einem medizinischen Gerät medizinische Daten erfasst, abrufbar gespeichert und an einen Empfänger übertragen werden, bei dem in einem Server die von dem medizinischen Gerät erfassten Daten abrufbar gespeichert werden, bei dem in wenigstens einem mit dem Server wenigstens zeitweise in Verbindung stehenden Client die von dem medizinischen Gerät erfassten Daten gespeichert und mittels einer auf dem Client befindlichen Auswerteapplikation eingesehen, aufbereitet, geändert oder ergänzt werden und bei dem die aufbereiteten, geänderten oder ergänzten Daten von dem Client an den Server übertragen und in dem Server gespeichert werden. Vorteilhafte Ausgestaltungen des Verfahrens sind Gegenstand der Unteransprüche.

Weitere Einzelheiten und Vorteile der Erfindung werden anhand eines in den Zeichnungen dargestellten Ausführungsbeispiels näher erläutert. Es zeigen:
- Fig. 1:: ein Datenfluss-Diagramm des Kommunikationssystems
- Fig. 2:: eine schematische Übersicht über mögliche Kommunikationspartner eines Defibrillators,
- Fig. 3:: eine schematische Darstellung der Einbindung der Defibrillatordaten in ein Klinik-Informationssystem,
- Fig. 4:: eine schematische Darstellung der Dateien des Servers und deren Beziehung zueinander,
- Fig. 5:: eine schematische Darstellung des Weiterversandes von Serverdaten und
- Fig. 6:: eine schematische Darstellung der Synchronisationstechnik des Kommunikationssystems.

Grundlegend für das erfindungsgemäße Kommunikationssystem und Verfahren ist das Prinzip, dass Daten des medizinischen Gerätes, beispielsweise eines Defibrillators, nicht direkt zu einer Bearbeitungsstation beispielsweise in der Klinik gesendet werden müssen, sondern auf einem zentralen Server abgelegt werden, von dem aus eine schnelle Verteilung an verschiedene Empfänger in verschiedenen Formen stattfinden kann. Vom Server können die Daten mittels der Clients abgeholt werden. Diese Kommunikation findet vorzugsweise über eine verschlüsselte HTTPS-Verbindung statt. Grundsätzlich ist auch denkbar, die Daten des Servers, beispielsweise die Einsatzdaten per Faxabruf vom Server anzufordern. Auch der Abruf oder das Übersenden von Serverdaten auf ein mobiles Endgerät, beispielsweise ein Mobiltelefon oder ein PDA sind denkbar.

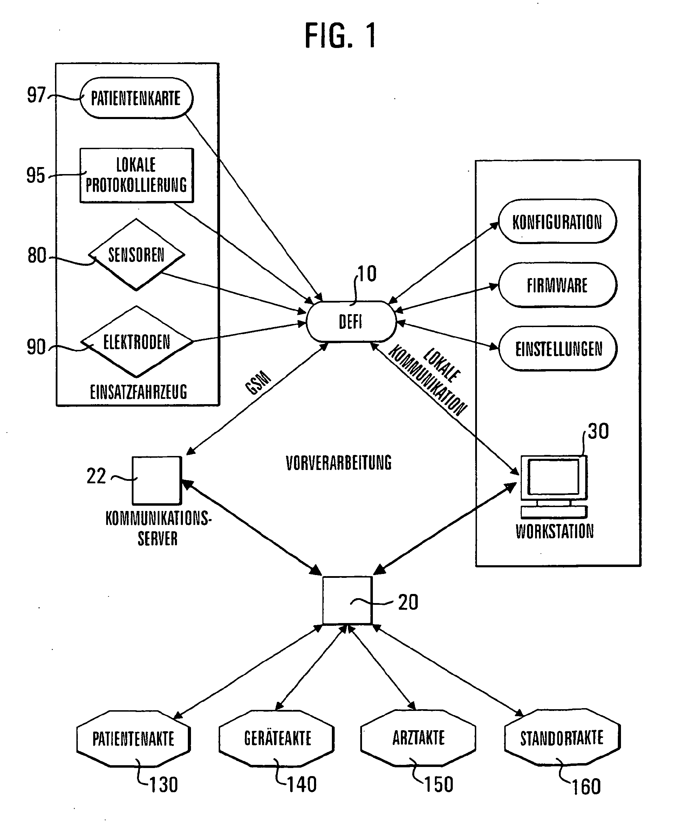

Fig. 1 zeigt den externen Defibrillator 10, der Daten von dessen Elektroden 90, externen Sensoren 80 im Nahbereich vorzugsweise mittels drahtloser Sensorik sowie von einer Protokollierungseinheit 95 des Defibrillators 10 über lokale Eingaben des Benutzers empfängt. Von einem lokalen Client 30 kann der Defibrillator 10 ferner vorzugsweise per Infrarot mit Standort-Konfigurationsdaten, Firmware-Updates und Geräteeinstellungen versorgt werden.

Die Standortkonfigurationsdaten werden vom Server 20 geladen, im Client 30 gegebenenfalls bearbeitet und sodann zum Defibrillator 10 gesendet. Grundsätzlich ist es ebenso möglich, diese Daten unmittelbar, beispielsweise per GSM, GPRS, UMTS etc. vom Server 20 an den Defibrillator 10 zu senden.

Entsprechendes gilt für Geräteeinstellungen des Defibrillators 10, die entweder über den Client 30 an den Defibrillator 10 übertragen werden oder unmittelbar vom Server 20 übertragen werden. Bei der Datenübertragung von Client 30 besteht die Möglichkeit, dass der Client 30 die Daten vom Server 20 abfragt oder mittels Synchronisation mit dem Server 20 vervollständigt.

Wie aus Fig. 1 ersichtlich erfolgt die Kommunikation mit dem Server 20 über den Kommunikationsserver 22.

Wie aus Fig. 1 weiter hervorgeht, können Basisinformationen zum Patienten von der Versichertenkarte 97 eingelesen werden. Alle darüber hinausgehenden Informationen können auf dem Defibrillator 10 eingegeben oder im Nachhinein durch den Client 30 während einer Nachbearbeitung erfasst werden, wie z.B. Freitexteingaben. Grundsätzlich ist es ebenso denkbar und vorteilhaft, wenn Patientendaten, beispielsweise bisherige Behandlungsdaten, Daten über Vorerkrankungen und dergleichen unmittelbar vom Server 20 an den Defibrillator 10 gesendet werden, damit diese bereits vor oder spätestens bei der Behandlung zur Verfügung stehen.

Die lokale Protokollierung 95 gemäß Fig.1 dient der Möglichkeit, bereits während des Einsatzes Daten einzugeben, die später zum Einsatzprotokoll vervollständigt werden können. Dies bezieht sich vor allem auf zeitkritische Angaben, wie die Protokollierung der Abgabe von Schocks durch den Defibrillator, die Gabe von Medikamenten oder die Einleitung therapeutischer Maßnahmen. Vorzugsweise wird ein Großteil dieser Maßnahmen und Eingaben standortweit in der Standortakte 160 des Servers 20 hinterlegt, so dass die entsprechenden möglichen Eingaben nur aus Listen auszuwählen sind. Die von den Sensoren 80 oder den Elektroden 90 erfassten Daten werden unmittelbar in den Defibrillator 10 eingelesen. Im Unterschied zu anderen erfassten Daten, haben alle Messwerte eine konstante Abtastfrequenz und können somit in Form eines Arrays platzsparend abgelegt werden. Es findet eine Zwischenspeicherung der einzelnen Kanäle im Speicher des Defibrillators 10 statt. Beim Auslesen der Daten durch den Client 30 oder Übersenden der Daten auf den Server 20 können dann die einzelnen Kanäle getrennt abgerufen werden. Somit kann auch ein Online-Streaming der Daten auf einen PC oder externen Monitor stattfinden.

Kontinuierliche Messwerte werden schon vor der Übertragung in EDF-Form verpackt und dann blockweise versandt. Somit befinden sich alle erfassten Kanäle in einer Übertragungseinheit. Die Übertragung muss in Echtzeit geschehen, so dass ein angeschlossener Monitor die Daten live wiedergeben kann. Beim nachträglichen Auslesen der Daten spielt die Echtzeitübertragung keine Rolle. Deshalb kann hier das Signal in der ganzen aufgezeichneten Qualität versandt werden. Patientendaten werden, soweit sie beim Einsatz erfasst werden, in dem Einsatzdatensatz verpackt übertragen. Eine Zuordnung von Patientendaten zu einer Patientenakte 130 findet auf dem Defibrillator 10 nicht statt, sondern erst im Nachhinein auf dem Client 30 während der Aufarbeitung. Die Patientendaten werden aus Gründen des Datenschutzes zwar in der Arztakte 150 des Servers 20, nicht jedoch weiter in die Standortakte 160 übernommen.

Zur Ablage der Daten kommuniziert der Defibrillator 10 mit dem Server 20 bzw. mit dem Kommunikationsserver 22 oder indirekt über den Client 30, der die Daten ggf. nach einer Aufbereitung oder nach Ergänzungen auf dem Server 20 ablegt. Dort werden die Inhalte separiert und der aus Fig. 1 ersichtlichen Patientenakte 130, der Geräteakte 140, der Arztakte 150 und der Standortakte 160 zugeordnet.

Grundsätzlich ist es ebenfalls möglich, die Kommunikation per Daten-Streaming vorzunehmen, bei dem alle vorhandenen Signale, inklusive per Bluetooth empfangene externe Signale als EDF-Stream über eine serielle Verbindung ausgegeben werden.

Jeder Arzt, der mit dem Kommunikationssystem arbeitet, erhält eine eigene Arztakte 150, in der seine persönlichen Daten abgelegt werden können. Derartige arztspezifische Daten umfassen: persönliche Daten des Arztes, administrative Daten des Arztes (Standort, Facharzt, etc.), Daten seiner Einsätze, persönliche Kommentare zu seinen Einsätzen, persönliche Abrechnungsdaten zu den Einsätzen, persönliche Defibrillatoren-Einstellungen.

Jeder Defibrillator 10, der einem Standort zugeordnet ist, erhält über die Standortakte 160 Zugriff auf die gemeinsamen Daten aller Defibrillatoren eines Einsatz-Standortes. Somit kann gewährleistet werden, dass sämtliche Defibrillatoren eines Standortes auf gemeinsame Vorgaben zugreifen. Des Weiteren werden die Einsatzdaten in der Standortakte 160 abgelegt, was den Vorteil mit sich bringt, dass ein Suchen nach Einsätzen auf regionaler Ebene ermöglicht wird ohne dass bekannt sein muss, welcher Defibrillator eingesetzt wurde. In der Standortakte 160 abgelegte, standortspezifische Daten sind: mögliches Bedienpersonal, um bei der Protokollierung schnell eine Listenauswahl treffen zu können, Liste der Defibrillatoren am Standort, Kliniken des Standortes mit Kontaktinformationen, standardisiertes Medikamenten-Repertoire des Standortes bzw. der Rettungswagen, Einsätze, die mit den Defibrillatoren des Standortes erbracht wurden.

In der die gerätespezifischen Daten enthaltenden Geräteakte 140 werden folgende Daten dokumentiert: Modell, Seriennummer, Standort, Eigentümer, Ansprechpartner, verantwortlicher Techniker, Softwareversion aller Komponenten, Anzahl der Einsätze, Anzahl der abgegebenen Schocks, nächster Wartungstermin, Wartungsprotokolle, Link zu der Standortakte 160.

Die Patientendaten, die während des Einsatzes protokolliert werden, werden vorläufig in der Arztakte 150 abgelegt. Von dort aus kann der Arzt die Daten in die Patientenakte 130 verschieben. Erfasst werden: Stammdaten von der Versichertenkarte, Notfallzeitpunkt, DIVI-Protokoll des Einsatzes, behandelnder Arzt.

Die Einsatzdaten werden wie oben ausgeführt in der Standortakte 160 abgelegt. Zusätzlich wird ein Einsatzprotokoll abgelegt, das den erforderlichen Standards entspricht. Dieses Protokoll wird teilweise zeitnah während des Einsatzes aufgezeichnet, kann jedoch auch nach dem Einsatz vom Arzt mit Hilfe des Clients 30 ergänzt werden. Die abgelegten Einsatzdaten dienen dem Arzt als Grundlage für die Erstellung eines Abrechnungsbogen. Die Inhalte des Einsatzdatensatzes sind: Notfallzeitpunkt, Notfallort, Abgabe des Notrufes, Eintreffen des Defibrillators 10, Eingeleitete Maßnahmen (Medikamente, Beatmung, Reanimation, Schockgabe) mit Zeitstempel, aufgezeichnete Messwerte (EKG, andere Sensoren), aufgezeichnete Patientendaten (von der Patientenkarte), Bedienpersonal des Defibrillators, Klinik der Nachbehandlung, Einweisungszeitpunkt, andere Angaben laut DIVI-Protokoll.

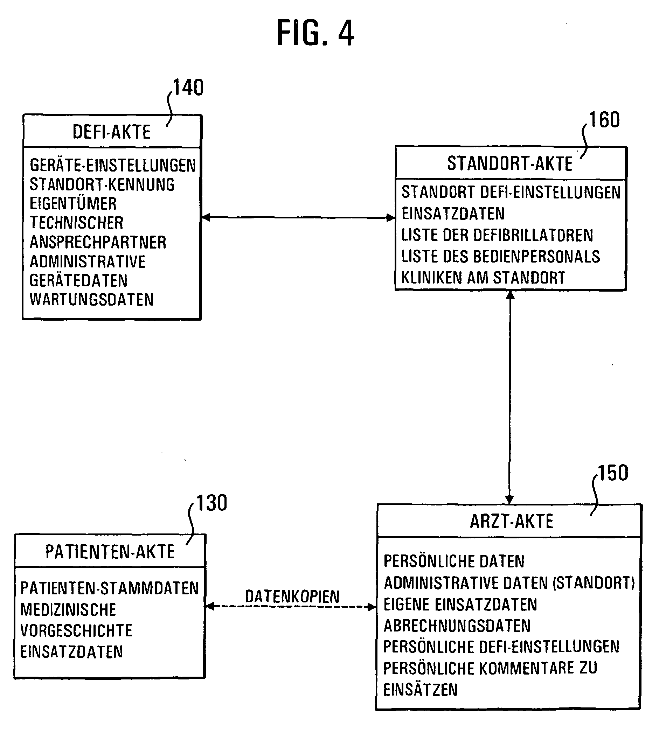

Fig. 4 zeigt die Beziehung der genannten Akten, d.h. Speicherbereiche 130-160 untereinander sowie deren bevorzugte Inhalte. Zu der Patientenakte 130 bestehen keine direkten Verbindungen, da dies aus datenschutzrechtlichen Gründen nicht zulässig wäre.

Es besteht die Möglichkeit, Teile der Daten in den verschiedenen Akten digital zu signieren. Dies kann durch eine Signatur von Teilbäumen des auf dem Server 20 abgelegten XML-Dokumentes realisiert werden. Somit können auch verschiedene Personen oder Einheiten getrennt voneinander Teilmengen der Daten signieren. Dies ist insbesondere in der Standortakte 160 relevant, in der verschiedene Ärzte Einträge vornehmen, die zweifelsfrei einer Person zuzuordnen sein müssen. Die Signatur gewährleistet hierbei die Integrität und die Nicht-Abstreitbarkeit der Daten. Die Signatur basiert auf einer Private-/Public-Key-Signatur auf Basis von GnuPG. Wer Daten im System signieren möchte, muss sich zuerst einen Schlüssel generieren lassen, von dem der öffentliche Schlüssel auf einem öffentlichen Key-Server abgelegt wird. Der private (geheime) Schlüssel bleibt im Eigentum des Besitzers und wird zusätzlich mit einer PIN gesichert. Zum Signieren von Daten wird aus dem zu signierenden XML-Fragment ein Hashwert gebildet, der mit dem privaten Schlüssel signiert wird. Der signierte Hashwert wird mit der Key-ID des Schlüssels an das XML-Dokument angehängt. Die Eindeutigkeit der Signatur gewährleistet, dass nur mit einem einzigen, bestimmten Schlüssel aus dem Hash-Wert der Daten die errechnete Signatur entstehen kann. Mit jedem anderen Schlüssel wäre eine andere Signatur entstanden. Zum Überprüfen der Signatur wird vom Key-Server der öffentliche Schlüssel mit der angegebenen Key-ID abgerufen und der inverse Algorythmus der Signatur auf die Daten angewandt. Hierbei muss der gleiche Wert entstehen wie bei der Signatur. Sind diese Werte nicht identisch, wurden die signierten Daten verändert und die Signatur ist ungültig.

In bevorzugter Weiterbildung der Erfindung findet eine Verschlüsselung von Daten statt. Dabei ist vorgesehen, dass die Verschlüsselung vorzugsweise an zwei Stellen stattfindet: auf der Kommunikationsstrecke, um ein Abhören der Daten zu verhindern sowie bei der Ablage in dem Server 20. Die Verschlüsselung auf der Kommunikationsstrecke wird vom HTTPS-Protokoll übernommen, das auf einer asymmetrischen SSL-Verschlüsselung aufbaut. Die Verschlüsselung zur Ablage in der Datenbank ist eine symmetrische, die nur eine zusätzliche Sicherung der Serverinfrastruktur darstellt. Eine Verschlüsselung der Daten für spezielle Empfänger, wie sie bei einer verschlüsselten Emailkommunikation stattfinden würde, findet nicht statt. Diese ist nicht erforderlich, da eine Prüfung der Zugriffsrechte bereits auf dem Server 20 stattfindet. Eine Verschlüsselung der Daten für einen bestimmten Empfänger kann dann relevant werden, wenn Einsatzdaten per Email an einen Empfänger versandt werden sollen.

Mittels des Clients 30 ist die Steuerung des Defibrillators 10 sowie die Verwaltung einer Vielzahl von medizinischen Geräten sowie die medizinische Bearbeitung der Daten möglich. Der Client 30 weist eine Applikation in Form einer JavaWebStart-Anwendung auf, was die Anwendung unabhängig vom Betriebssystem des Anwenders macht.

Die meisten anfallenden medizinischen Daten werden in Form eines XML-Dokumentes verpackt. In dieser Form können die Daten sowohl von der Client-Software als auch vom Server 20 verarbeitet und visualisiert werden. Für die aufgezeichneten Messreihen eignet sich jedoch das EDF-Format besser, da bei dessen Verwendung deutlich weniger Overhead anfällt als beim Einsatz von XML. Die Verbindung zwischen XML- und EDF-Daten wird über eine Verlinkung des EDF-Dokumentes aus dem XML-Dokument heraus erreicht.

Der Kommunikationsserver 22 gemäß Fig. 1 dient dazu, die Übertragung von Daten vom Defibrillator 10 auf den Server 20 oder in umgekehrter Richtung zu ermöglichen. Der Server 22 empfängt die gleichen Datenpakete wie der Client 30 sie per Infrarot empfängt. Hieraus wird das Einsatzprotokoll in XML-Form sowie getrennt davon die EDF-Datei mit den Messwerten generiert. Beides wird umgehend auf den Server 20 übertragen. Der Server 22 kann direkt auf dem Server 20 realisiert werden. Die Übertragung der Daten findet beispielsweise per V.110-Schnittstelle über das GSM-Netz und somit digital statt.

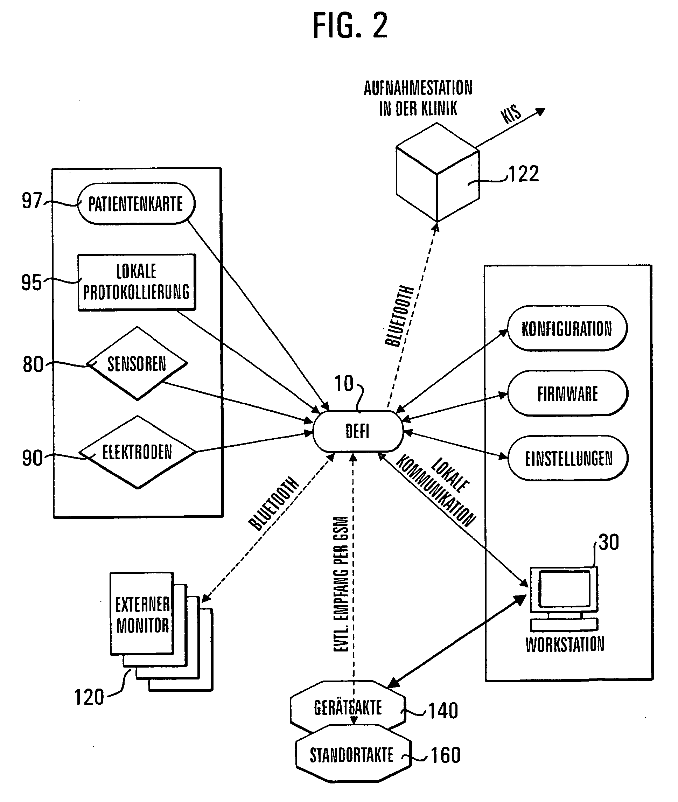

Fig. 2 zeigt in einer weiteren schematischen Darstellung die Kommunikationspartner des Defibrillators 10. Neben den in Fig. 1 bereits dargestellten Kommunikationspartnern ist in Fig. 2 der oder die externen Monitore 120 vorgesehen, die Messwerte in Echtzeit per Bluetooth erhalten.

Des weiteren ist in Fig. 2 eine Aufnahmestation einer Klinik schematisch dargestellt. Diese weist eine Empfangseinheit 122 auf, die über eine Kommunikationsschnittstelle zum Empfang der in dem Defibrillator 10 gespeicherten Daten mittels Bluetooth verfügt.

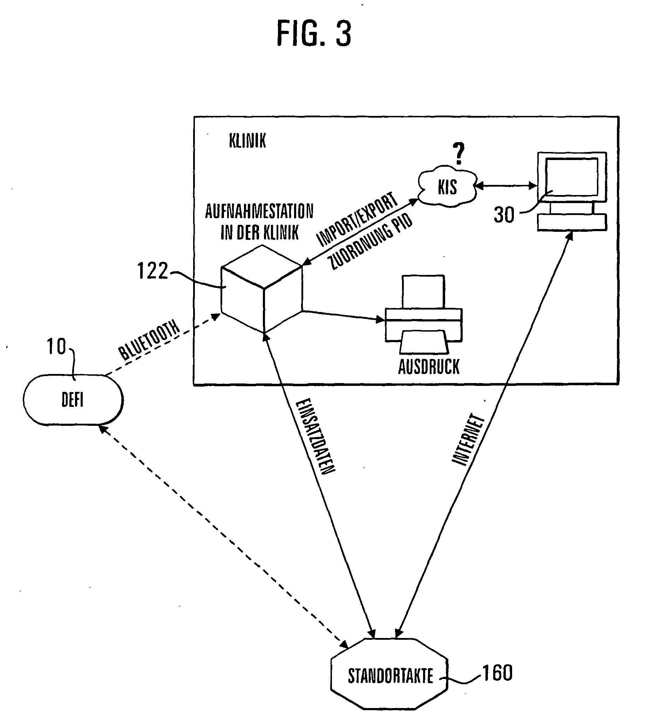

Fig. 3 zeigt schematisch, wie die Einsatzdaten der Klinik für eine Nachbehandlung zur Verfügung gestellt werden können. Neben dem unmittelbaren Empfang der Daten von dem Defibrillator 10 besteht die Möglichkeit, über einen Internetanschluss auf die Einsatzdaten der Standortakte 160 des Servers 20 zuzugreifen. Alternativ dazu können Einsatzdaten unmittelbar von der Empfangseinheit 122 aus der Standortakte 160 bezogen werden, wie dies aus Fig. 3 hervorgeht. Dies setzt jedoch voraus, dass die Daten vom Defibrillator 10 rechtzeitig an den Server 20 und an dessen Standortakte 160 übertragen werden.

Des weiteren besteht die in Fig. 3 ersichtliche Möglichkeit, die Datensätze von der Empfangseinheit 122 mit den in der Klinik vorgenommenen Änderungen an den Server 20 zu senden. Eine weitere Möglichkeit besteht darin, den Datensatz in der Klinik über den Client 30 zu visualisieren und zu verarbeiten. Die Daten des Servers können drahtlos an den Rettungsdienst übertragen werden, was insbesondere hinsichtlich der Einsatzdaten früherer Einsätze von Vorteil ist. Grundsätzlich ist es auch möglich, vor Beginn der Fahrt die Daten auf den Client 30 zu replizieren und dann während der Fahrt zum Notfall-Einsatzort zu begutachten.

Folgende Funktionalitäten sind in der Empfangseinheit 122 implementiert: Empfang von Einsatzdaten inklusive Patienteninformationen vom Defibrillator 10 mittels Bluetooth; Empfang der Daten von der Standortakte 160; sofortiger Ausdruck des Einsatzprotokolls; Browser-basierte Administration der früher empfangenen und aktuellen Einsätze. Dabei sollten folgende Funktionalitäten implementiert sein: Einsatzdaten in die KIS-interne Dokumentation übernehmen; Einsatzdaten einer vorhandenen Patientenakte zuweisen; Anlegen einer neuen Patientenakte aufgrund des Vorfalls; Übernahme der KIS-Dokumentation in die Patientenakte.

Eine Datenverarbeitung findet auf dem Defibrillator 10, dem Client 30, auf dem Server 20 sowie auch in der Empfangseinheit 122 statt. Im Client 30 werden die auf dem Defibrillator 10 erfassten Daten nochmals verifiziert und zum vollständigen DIVI-Protokoll ergänzt. Im Client 30 können die erfassten EKG-Daten nochmals durch entsprechende Algorithmen ausgewertet und klassifiziert werden, soweit dies nicht schon umfassend auf dem Defibrillator 10 geschehen ist. Die errechnete Klassifikation kann anschließend direkt im EKG hinterlegt werden. Die EKG-Darstellung im Client 30 erlaubt ein schnelles Befunden innerhalb des Datensatzes. Auffällige Stellen können durch Mausbewegungen ausgewählt, gezoomt und vermessen werden. Durch einfachen Doppelklick in EKG kann an der entsprechenden Stelle ein Vermerk in den Datenverlauf eingefügt werden, der dann in den Einsatzdaten abgelegt wird.

Das EKG kann in dieser Darstellung auch in Echtzeit, synchron zu den aufgezeichneten Audiodaten wiedergegeben werden. Hauptaufgabe des Clients 30 in der Arztversion wird es sein, das Einsatzprotokoll zu vervollständigen. Die durch die lokale Protokollierung erfassten Daten werden nachbearbeitet und ergänzt. Texteingaben sind ergonomisch möglich.

Darüber hinaus ermöglicht der Client 30 die Erstellung einer Einsatzabrechnung in Form einer Rechnung.

Sofern Patienteninformationen nicht bereits während des Einsatzes von der Patientenkarte eingelesen werden konnten, können diese mittels des Clients 30 nachgetragen werden. Besonders zu berücksichtigen ist, dass eine Möglichkeit bestehen sollte, die Einsatzdaten einer vorhandenen Patientenakte 130 zuzuordnen oder eine neue Patientenakte 130 zu generieren, die sodann mit den Einsatzdaten gefüllt wird.

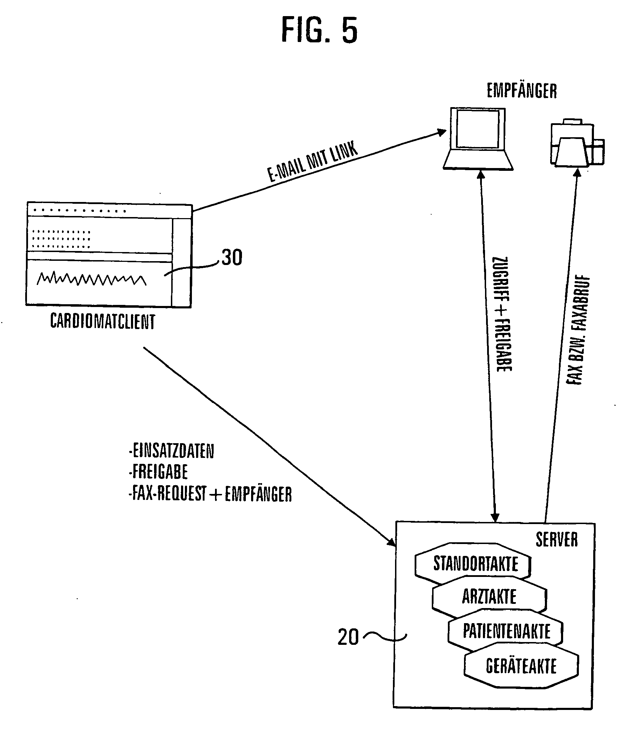

Fig. 5 zeigt den Weiterversand der auf dem Server 20 gespeicherten Daten. Der Weiterversand ermöglicht eine rasche Weitergabe von aufgezeichneten Einsatzdaten an bestimmte Empfänger. Folgende Mechanismen stehen zur Verfügung:

| | |
|---|---|
| Email mit Link: | Der Versender gibt den Datensatz in der Standortakte 160 frei und schickt dem Empfänger einen Link, der direkt diesen Datensatz im Browser anzeigt. |
| | |
| Faxversand: | Der Versender weist den Server 20 an, ein Fax mit dem Einsatzprotokoll an einen definierten Empfänger zu senden. Es können auch automatisch Einsatzprotokolle an alle Kliniken im Standortbereich versandt werden. |
| | |
| Faxabruf: | Es können einer Standortakte 160 Fax-Abruf-Kennungen zugeordnet werden, die ein direktes Abrufen des letzten Einsatzes per Fax-Polling vom Server erlauben. |
| | |
| Synchronisation: | Wenn ein Empfänger ebenfalls über einen Client 30 verfügt, kann ein Zugriff auf neue Einsätze durch einfache Synchronisation erfolgen, wie dies unten noch näher erläutert wird. |

Des weiteren ist es möglich, den Client 30 mit externen Datenquellen zu versorgen. Auch ist es möglich, in dem Client 30 statistische Auswertungen über die durchgeführten Einsätze vorzunehmen, was insbesondere für die Qualitätssicherung von Bedeutung ist.

Mögliche Empfänger von auf dem Server 20 gespeicherten Daten sind die Klinik, der Rettungsdienst, der Hausarzt und eine Rehabilitationseinrichtung, der Rettungsdienst zum Zwecke der Qualitätssicherung sowie der Patient.

Eine einfache Lösung hinsichtlich der Kommunikation mit der Klinik besteht darin, die Daten per Telefax von dem Server 20 in die Klinik zu übertragen. Hierzu muss der Defibrillator 10 ausgelesen und die Einsatzdaten müssen auf den Server 20 übertragen werden, was eine Verzögerung mit sich bringt. Hinsichtlich der Geschwindigkeit vorteilhafter ist der Empfang der Daten in der Klinik über die Empfangseinheit 122 beispielsweise per Bluetooth gemäß Fig. 3. Die Empfangseinheit 122 empfängt den Einsatzdatensatz, wie er während des Einsatzes mitprotokolliert wurde. Von der Empfangseinheit 122 können die Daten abgerufen oder ausgedruckt werden. Die Daten können anschließend am Bildschirm dargestellt werden oder unter Umständen direkt in das Klinik-Informationssystem importiert werden.

Hinsichtlich des Hausarztes und der Rehabilitationseinrichtungen spielt eine Latenzzeit zwischen Unfalleinsatz und der Verfügbarkeit der Daten eine untergeordnete Rolle. Der Datenzugriff kann einerseits über den Client 30 oder auch über eine Internetansicht erfolgen oder auch per Faxabruf vom Server angefordert werden.

Für den Patienten ist ebensowenig wie für den Hausarzt oder die Rehabilitationseinrichtung einer Echtzeitzugriff auf die Daten erforderlich. Hier ist es ausreichend, wenn der Patient, der die Daten vom Hausarzt, der Klinik oder vom Rettungsdienst in seine Patientenakte 130 kopiert bekommt, nach der Behandlung Zugriff auf die Daten hat. Dieser Zugriff kann per Web-Browser stattfinden, da keine Bearbeitungsfunktionalität erforderlich ist.

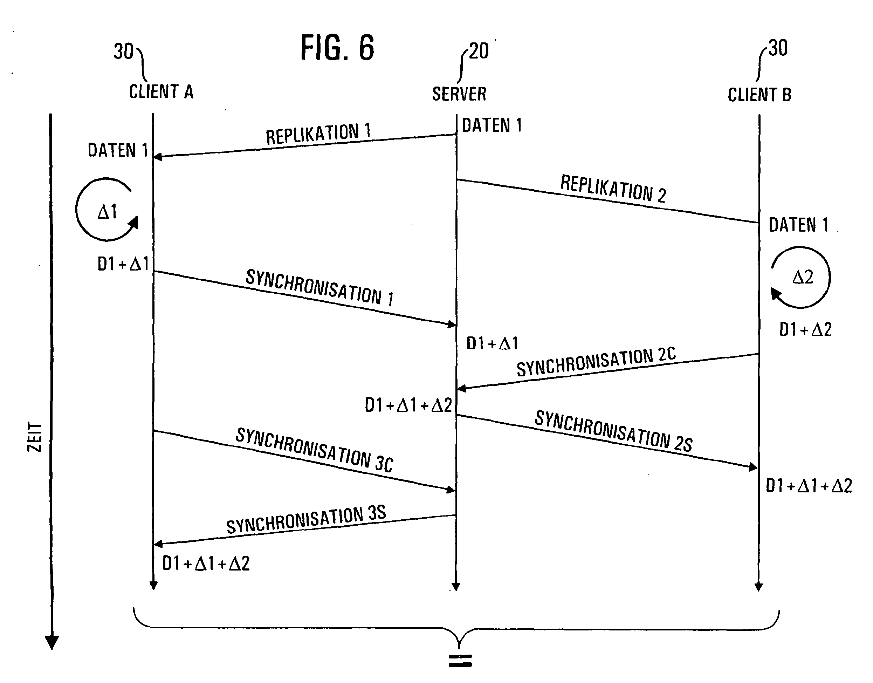

Fig. 6 zeigt den Ablauf der Synchronisation von Server- und Client-Daten. Wie aus Fig. 6 hervorgeht, werden Teile des XML-Datenobjektes des Servers 20 repliziert (Replikation 1, 2) und auf den Clients 30 (Client A, Client B) lokal gespeichert. Änderungen auf den Clients (Δ1, Δ2) werden protokolliert und beim Synchronisationsvorgang (Synchronisation 1, 2C) auf dem Server 20 nachvollzogen. Änderungen die in der Zwischenzeit durch andere Clients auf den Server synchronisiert wurden, werden nach einer entsprechenden Prüfung (Synchronisation 2C, 3C) wiederum an den synchronisierenden Client übergeben (Synchronisation 2S, 3S). Im Einzelnen umfasst die Synchronisation folgende Schritte: Protokollierung der Änderungen nach Xpath, Zeitpunkt und Art der Änderung auf dem Client; Übertragung der Änderungen auf den Server; Überprüfen der auf dem Server protokollierten Änderungen seit der letzten Synchronisation des Clients; Auflösung von Konflikten der Synchronisation; Ablegen der Änderungen in einer Tabelle auf dem Server; Übertragen der verbleibenden Änderungen an den Clients; wenn Binär-Elemente auf dem Client geändert oder hinzugefügt werden, werden diese geänderten Objekte vom Client angefordert; Ablegen der Binärobjekte in der Datenbank.

Auf dem Client neu angelegte Objekte, die mehrfach in einer Sammlung vorkommen können, werden auf dem Client mit temporären (negativen) IDs versehen. Während der Synchronisation wird dann auf dem Server kontrolliert, welche IDs für diesen Pfad bereits vergeben sind. Der Server ändert dann die Negative IDs zu nächst höheren noch nicht vergebenen ab. Diese Änderung wird dann auch dem Client mitgeteilt, damit dieser seine lokale Kopie aktualisieren kann. Auf diese Weise können verschiedene Clients gleichzeitig neue Elemente hinzufügen ohne sich gegenseitig zu beeinflussen.

Von Bedeutung kann es sein, dass bei Erreichen oder Überschreiten von Daten-Grenzwerten Aktionen ausgelöst werden können. Hierzu werden vorzugsweise sogenannte Watch-Points gesetzt, die bei Überschreitung eines Grenzwertes eine Meldung, beispielsweise per SMS, abgeben. Dabei sind folgende Schritte vorzunehmen: Definieren des zu beobachtenden Daten-Elementes; Definieren der zu beachtenden Kriterien; Definieren der auszulösenden Aktion (Versand einer Nachricht, etc.); Ablegen des Watch-Points in der Datenbank; Abarbeitung aller Watch-Points einer Akte bei jeder Synchronisation; Zeitgesteuerte Abarbeitung aller Watch-Points aller Akten.

Um eine sichere Datenverwaltung zu gewährleisten, kann es von Vorteil sein, verschiedenen Nutzern Freigaben auf verschiedene Teile des Datensatzes des Servers 20 zu erteilen. Hierbei kann festgelegt werden, welcher Benutzer auf welchem Teilbaum des XML-Datenobjektes lesend und schreibend zugreifen darf. Des weiteren kann ein Nutzer die Administrationsrechte an seinem eigenen Datensatz an andere Nutzer weitergeben, die dann quasi als Stellvertreter das Rechtemanagement des Eigentümers übernehmen. Für den Nutzer sind die Pfade in XML-Baum hinter textuellen Beschreibungen versteckt. Folgende Schritte sind dabei durchzuführen: Auswahl oder Anlegen eines Mitbenutzers des eigenen Datensatzes; Festlegung der Schreib-/Leserechte auf dem Datensatz; eventuell Definition des Mitbenutzers als Administrator auf dem eigenen Datensatz, der dann wiederum weitere Mitbenutzer (und auch Mit-Administratoren) anlegen kann.

Dabei besteht die Möglichkeit, Rechte nicht nur auf einzelne Elemente der Daten vergeben zu können, sondern auf gesamte Themen-Komplexe, die sich an verschiedenen Orten des Datenobjektes des Servers befinden können.

## Patentansprüche

1. Kommunikationssystem zur Bearbeitung medizinischer Daten,
das Kommunikationssystem umfassend ein medizinisches Gerät, das Erfässungsmittel zur Erfassung medizinischer Daten, eine Speichereinheit zur abrufbaren Speicherung der Daten sowie eine Kommunikationsschnittstelle zur Datenübergabe aufweist,
einen Server (20), der eine Speichereinheit, in der die von dem medizinischen Gerät erfaßten Daten abrufbar gespeichert sind, sowie eine Kommunikationsschnittstelle aufweist, mittels derer der Server (20) mit einem oder mehreren Clients (30) wenigstens zeitweise in Verbindung steht,
sowie mehrere Clients (30), die eine Auswerteeinheit sowie eine Kommunikationsschnittstelle aufweisen,
**dadurch gekennzeichnet,**
**dass** die Clients (30) ferner eine Speichereinheit aufweisen, in der die von dem medizinischen Gerät erfaßten Daten gespeichert sind, wobei die Auswerteeinheit eine Auswerteapplikation zur Einsicht, Aufbereitung, Änderung oder Ergänzung der in der Speichereinheit der Clients (30) gespeicherten Daten umfaßt, wobei mittels der Kommunikationsschnittstelle die in den Clients (30) aufbereiteten, geänderten oder ergänzten Daten von den Clients (30) an den Server (20) übermittelbar sind und wobei diese Daten in der Speichereinheit des Servers (20) speicherbar sind,
**dass** Mittel zur Synchronisation von Daten des Servers (20) und der Clients (30) vorgesehen sind, mittels derer ausschließlich in einem Client (30) aufbereitete, geänderte oder ergänzte Daten an den Server (20) übermittelbar sind und
**dass** Mittel vorgesehen sind, mittels derer die durch weitere Clients (30) vorgenommene und auf dem Server (20) gespeicherte Aufbereitungen, Änderungen oder Ergänzungen von Daten von dem Server (20) an die Clients (30) übertragen werden, so dass in sämtlichen Clients (30) und in dem Server (20) identische Datensätze vorliegen.

2. Kommunikationssystem nach Anspruch 1, **dadurch gekennzeichnet, dass** die in dem medizinischen Gerät gespeicherten Daten über dessen Kommunikationsschnittstelle unmittelbar an den oder die Clients (30) oder an den Server (20) übermittelbar sind.

3. Kommunikationssystem nach Anspruch 1 oder 2, **dadurch gekennzeichnet, dass** es sich bei dem medizinischen Gerät um einen externen Defibrillator (10) handelt.

4. Kommunikationssystem nach einem der vorhergehenden Ansprüche, **dadurch gekennzeichnet, dass** die durch weitere Clients vorgenommenen und auf dem Server (20) gespeicherten Aufbereitungen, Änderungen oder Ergänzungen von Daten von dem Server (20) an den Client (30) übertragbar sind.

5. Kommunikationssystem nach einem der vorhergehenden Ansprüche, **dadurch gekennzeichnet, dass** es sich bei der auf dem Client (30) befindlichen Auswerteapplikation um eine Java WebStart Anwendung handelt.

6. Kommunikationssystem nach einem der vorhergehenden Ansprüche, **dadurch gekennzeichnet, dass eine Kommunikationsschnittstelle des medizinischen** Gerätes als Mobilfunkschnittstelle ausgeführt ist und dass der Server (20) oder ein mit diesem in Verbindung stehender Kommunikationsserver (22) ebenfalls über eine Mobilfunkschnittstelle verfügt, mittels derer Daten von dem medizinischen Gerät an den Server (20) oder an den Kommunikationsserver (22) und von diesem an den Server (20) übertragbar sind.

7. Kommunikationssystem nach Anspruch 6, **dadurch gekennzeichnet, dass** der Kommunikationsserver (22) Bestandteil des Servers (20) ist.

8. Kommunikationssystem nach einem der vorhergehenden Ansprüche, **dadurch gekennzeichnet, dass** eine Kommunikationsschnittstelle des medizinisches Gerätes als Mobilfunkschnittstelle ausgeführt ist und dass der Server (20) oder ein mit diesem in Verbindung stehender Kommunikationsserver (22) ebenfalls über eine Mobilfunkschnittstelle verfügt, mittels derer Daten von dem Server (20) an das medizinische Gerät oder von dem Server (20) an den Kommunikationsserver (22) und von diesem an das medizinische Gerät übertragbar sind.

9. Kommunikationssystem nach einem der vorhergehenden Ansprüche, **dadurch gekennzeichnet, dass** in der Speichereinheit des Servers (20) oder des Clients (30) für den Standort des medizinischen Gerätes spezifische Daten, Firmware-Updates, Daten betreffend Geräteeinstellungen des medizinischen Gerätes oder Patientendaten, insbesondere Vorerkrankungen, aktuelle oder vergangene Behandlungen betreffende Daten abgelegt sind, und dass diese Daten oder Updates mittels der Kommunikationsschnittstellen des Servers (20), des Clients (30) und des medizinischen Gerätes unmittelbar vom Server (20) oder vom Client (30) an das medizinische Gerät oder vom Server (20) an den Client (30) und mittels der Kommunikationsschnittstellen des Clients (30) und des medizinischen Gerätes vom Client (30) zum medizinischen Gerät übertragbar sind.

10. Kommunikationssystem nach einem der vorhergehenden Ansprüche, **dadurch gekennzeichnet, dass** das medizinische Gerät über Mittel zur Erfassung von im Rahmen einer Behandlung angefallenen Messwerten verfügt, wobei die Mittel zur Messwerterfassung Detektoren umfassen, mittels derer von Sensoren (80) per Infrarot oder Bluetooth übertragene Daten erfassbar sind.

11. Kommunikationssystem nach Anspruch 3 und 10, **dadurch gekennzeichnet, dass** die Mittel zur Messwerterfassung die Elektroden (90) des Defibrillators (10) umfassen.

12. Kommunikationssystem nach einem der vorhergehenden Ansprüche, **dadurch gekennzeichnet, dass** das medizinische Gerät Mittel zur Erfassung von Patientendaten aufweist.

13. Kommunikationssystem nach einem der vorhergehenden Ansprüche, **dadurch gekennzeichnet, dass** das medizinische Gerät Mittel (95) zur Eingabe und/oder zur Auswahl von Daten aufweist.

14. Kommunikationssystem nach einem der vorhergehenden Ansprüche, **dadurch gekennzeichnet, dass** das medizinische Gerät über eine Kommunikationsschnittstelle verfügt, mittels derer im Rahmen einer Behandlung angefallene Messwerte in Echtzeit an einen Monitor (120) übertragbar sind.

15. Kommunikationssystem nach Anspruch 14, **dadurch gekennzeichnet, dass** die Messwerte über eine Kommunikationsschnittstelle des medizinischen Gerätes mittels Bluetooth übertragbar sind.

16. Kommunikationssystem nach einem der vorhergehenden Ansprüche, **dadurch gekennzeichnet, dass** das Kommunikationssystem eine Empfangseinheit (122) mit einer Kommunikationsschnittstelle zum Empfang der in dem medizinischen Gerät abrufbar gespeicherten Daten mittels Infrarot oder Bluetooth aufweist.

17. Kommunikationssystem nach Anspruch 16, **dadurch gekennzeichnet, dass** die Empfangseinheit (122) ferner eine Schnittstelle zum Datenaustausch mit dem Server (20) aufweist.

18. Kommunikationssystem nach einem der vorhergehenden Ansprüche, **dadurch gekennzeichnet, dass** das medizinische Gerät einen integrierten Monitor zur Visualisierung erfasster Behandlungsdaten aufweist.

19. Kommunikationssystem nach einem der vorhergehenden Ansprüche, **dadurch gekennzeichnet, dass** der Server (20) über mehrere Datensätze verfügt, von denen wenigstens ein erster Datensatz (130) Patientendaten und wenigstens ein zweiter Datensatz (140) das medizinische Gerät betreffende Daten umfasst, wobei die in dem zweiten Datensatz (140) abgelegten Daten keine Zuordnung zu einem bestimmten Patienten aufweisen.

20. Kommunikationssystem nach Anspruch 19, **dadurch gekennzeichnet, dass** der Server (20) wenigstens einen dritten Datensatz (150) mit für einen Arzt spezifischen Daten und wenigstens einen vierten Datensatz (160) mit für den Standort des medizinischen Gerätes spezifischen Daten umfasst

21. Kommunikationssystem nach einem der vorhergehenden Ansprüche, **dadurch gekennzeichnet, dass** der Server (20) über eine Kommunikationsschnittstelle verfügt, mittels derer Daten von per Telefax übermittelten Dokumenten in den Server (20) einlesbar sind.

22. Kommunikationssystem nach einem der vorhergehenden Ansprüche, **dadurch gekennzeichnet, dass** der Server (20) über eine Kommunikationsschnittstelle verfügt, mittels derer auf dem Server (20) gespeicherte Daten per Telefax übermittelbar sind.

23. Kommunikationssystem nach einem der vorhergehenden Ansprüche, **dadurch gekennzeichnet, dass** in der Speichereinheit des Servers (10) mit den darin abgelegten Daten verknüpfte Bedingungen oder Grenzwerte abgelegt sind, und dass Mittel vorgesehen sind, mittels derer bei Eintreten der Bedingung oder bei Erreichen des Grenzwertes eine Meldung oder Mitteilung generierbar ist.

24. Verfahren zur Bearbeitung medizinischer Daten, bei dem in einem medizinischen Gerät medizinische Daten erfaßt, abrufbar gespeichert und an einen Empfänger übertragen werden und bei dem in einem Server (20) die von dem medizinischen Gerät erfaßten Daten abrufbar gespeichert werden,
**dadurch gekennzeichnet,**
**dass** in mit dem Server (20) wenigstens zeitweise in Verbindung stehenden Clients (30) die von dem medizinischen Gerät erfaßten Daten gespeichert und mittels einer auf den Clients (30) befindlichen Auswerteapplikation eingesehen, aufbereitet, geändert oder ergänzt werden, dass die aufbereiteten, geänderten oder ergänzten Daten von den Clients (30) an den Server (20) übertragen und in dem Server (20) gespeichert werden,
**dass** zum Zwecke der Synchronisation von Daten ausschließlich in einem Client (30) aufbereitete, geänderte oder ergänzte Daten an den Server (20) übertragen werden
und **dass** die von weiteren Clients (30) vorgenommenen und auf dem Server (20) gespeicherten Aufbereitungen, Änderungen oder Ergänzungen von dem Server (20) an die Clients (30) übertragen werden, so dass in sämtlichen Clients (30) und in dem Server (20) identische Datensätze vorliegen.

25. Verfahren nach Anspruch 24, **dadurch gekennzeichnet, dass** die in dem medizinischen Gerät gespeicherten Daten von dem medizinischen Gerät unmittelbar an den Server (20) oder an den Client (30) übertragen werden.

26. Verfahren nach Anspruch 24 oder 25, **dadurch gekennzeichnet, dass** es sich bei dem medizinischen Gerät um einen externen Defibrillator (10) handelt.

27. Verfahren nach einem der vorhergehenden Ansprüche, **dadurch gekennzeichnet, dass** die Datenübertragung zwischen medizinischem Gerät und dem Client (30) per Infrarot-Datenübertragung erfolgt.

28. Verfahren nach einem der vorhergehenden Ansprüche, **dadurch gekennzeichnet, dass** Daten mittels Mobilfunktechnik von dem medizinischen Gerät an den Server (20) oder an einen mit diesem in Verbindung stehenden Kommunikationsserver (22) oder von dem Server (20) oder dem Kommunikationsserver (22) an das medizinische Gerät übertragen werden.

29. Verfahren nach einem der vorhergehenden Ansprüche, **dadurch gekennzeichnet, dass** im Rahmen der Behandlung angefallene Messwerte von dem medizinischen Gerät erfasst und aufgezeichnet werden.

30. Verfahren nach Anspruch 26 und 29, **dadurch gekennzeichnet, dass** die Datenerfassung mittels der Elektroden (90) des Defibrillators (10) oder mittels Detektoren erfolgt, die die Daten per Infrarot- oder Bluetooth-Datenübertragung erhalten.

31. Verfahren nach einem der vorhergehenden Ansprüche, **dadurch gekennzeichnet, dass** eine Empfangseinheit (122) vorgesehen ist, und dass die in dem medizinischen Gerät gespeicherten Daten an die Empfangseinheit (122) per Infrarot oder Bluetooth übertragen werden.

32. Verfahren nach Anspruch 31, **dadurch gekennzeichnet, dass** Daten von dem Server (20) an die Empfangseinheit (122) und/oder von der Empfangseinheit (122) an den Server (20) übertragen werden.

33. Verfahren nach einem der vorhergehenden Ansprüche, **dadurch gekennzeichnet, dass** der Server (20) durch eine Telefaxnummer adressierbar ist und dass der Dateninhalt des Telefaxes in den Server (20) eingelesen wird.

34. Verfahren nach einem der vorhergehenden Ansprüche, **dadurch gekennzeichnet, dass** auf Anforderung des Empfängers oder eines Dritten in dem Server (20) abgelegte Daten per Telefax versendet werden.

## Claims

1. Communication system for editing medical data, the communication system comprising a medical appliance which has capture means for capturing medical data, a memory unit for retrievably storing the data and a communication interface for data transfer, a server (20) which has a memory unit, storing the data captured by the medical appliance in retrievable form, and a communication interface, by means of which the server (20) is at least intermittently connected to one or more clients (30), and also a plurality of clients (30) which have an evaluation unit and a communication interface,
**characterized**
**in that** the clients (30) also have a memory unit storing the data captured by the medical appliance, where the evaluation unit comprises an evaluation application for inspecting, conditioning, changing or adding to the data stored in the memory unit of the clients (30), where the communication interface can be used to transmit the data conditioned, changed or added to in the clients (30) from the clients (30) to the server (20), and where these data can be stored in the memory unit of the server (20),
**in that** means for synchronizing data from the server (20) and from the clients (30) are provided which can be used to transmit data conditioned, changed or added to exclusively in a client (30) to the server (20), and
**in that** means are provided which are used to transmit the conditionings, changes or additions performed on data by further clients (30) and stored on the server (20) from the server (20) to the clients (30), so that all clients (30) and the server (20) contain identical data records.

2. Communication system according to Claim 1, **characterized in that** the data stored in the medical appliance can be transmitted directly to the client(s) (30) or to the server (20) via said medical appliance's communication interface.

3. Communication system according to Claim 1 or 2, **characterized in that** the medical appliance is an external defibrillator (10).

4. Communication system according to one of the preceding claims, **characterized in that** the conditionings, changes or additions performed on data by further clients and stored on the server (20) can be transmitted from the server (20) to the client (30).

5. Communication system according to one of the preceding claims, **characterized in that** the evaluation application on the client (30) is a Java WebStart application.

6. Communication system according to one of the preceding claims, **characterized in that** a communication interface of the medical appliance is in the form of a mobile radio interface, and **in that** the server (20) or a communication server (22) connected thereto likewise has a mobile radio interface which can be used to transmit data from the medical appliance to the server (20) or to the communication server (22) and from the latter to the server (20).

7. Communication system according to Claim 6, **characterized in that** the communication server (22) is part of the server (20).

8. Communication system according to one of the preceding claims, **characterized in that** a communication interface of the medical appliance is in the form of a mobile radio interface, and **in that** the server (20) or a communication server (22) connected thereto likewise has a mobile radio interface which can be used to transmit data from the server (20) to the medical appliance or from the server (20) to the communication server (22) and from the latter to the medical appliance.

9. Communication system according to one of the preceding claims, **characterized in that** the memory unit of the server (20) or of the client (30) stores data specific to the location of the medical appliance, firmware updates, data relating to appliance settings for the medical appliance or patient data, particularly data relating to previous illnesses, current or past treatments, and **in that** these data or updates can be transmitted directly from the server (20) or from the client (30) to the medical appliance or from the server (20) to the client (30) using the communication interfaces of the server (20), of the client (30) and of the medical appliance and can be transmitted from the client (30) to the medical appliance using the communication interfaces of the client (30) and of the medical appliance.

10. Communication system according to one of the preceding claims, **characterized in that** the medical appliance has means for capturing measured values produced in the course of a treatment, the means for measured value capture comprising detectors which can be used to capture data transmitted from sensors (80) by infrared or Bluetooth.

11. Communication system according to Claims 3 and 10, **characterized in that** the means for measured value capture comprise the electrodes (90) of the defibrillator (10).

12. Communication system according to one of the preceding claims, **characterized in that** the medical appliance has means for capturing patient data.

13. Communication system according to one of the preceding claims, **characterized in that** the medical appliance has means (95) for inputting and/or selecting data.

14. Communication system according to one of the preceding claims, **characterized in that** the medical appliance has a communication interface which can be used to transmit measured values produced in the course of a treatment to a monitor (120) in real time.

15. Communication system according to Claim 14, **characterized in that** the measured values can be transmitted via a communication interface of the medical appliance using Bluetooth.

16. Communication system according to one of the preceding claims, **characterized in that** the communication system has a reception unit (122) having a communication interface for receiving the data stored in the medical appliance in retrievable form using infrared or Bluetooth.

17. Communication system according to Claim 16, **characterized in that** the reception unit (122) also has an interface for data interchange with the server (20).

18. Communication system according to one of the preceding claims, **characterized in that** the medical appliance has an integrated monitor for visually displaying captured treatment data.

19. Communication system according to one of the preceding claims, **characterized in that** the server (20) has a plurality of data records, from which at least a first data record (130) comprises patient data and at least a second data record (140) comprises data relating to the medical appliance, the data stored in the second data record (140) having no association with a particular patient.

20. Communication system according to Claim 19, **characterized in that** the server (20) comprises at least a third data record (150) having data specific to a doctor and at least a fourth data record (160) having data specific to the location of the medical appliance.

21. Communication system according to one of the preceding claims, **characterized in that** the server (20) has a communication interface which can be used to read data from documents transmitted by fax into the server (20).

22. Communication system according to one of the preceding claims, **characterized in that** the server (20) has a communication interface which can be used to transmit data stored on the server (20) by fax.

23. Communication system according to one of the preceding claims, **characterized in that** the memory unit of the server (20) stores conditions or limit values which are logically combined with the data stored therein, and **in that** means are provided which can be used to generate a report or announcement when the condition arises or when the limit value is reached.

24. Method for editing medical data, in which a medical appliance captures medical data, stores them in retrievable form and transmits them to a receiver and in which a server (20) is used to store the data captured by the medical appliance in retrievable form,
**characterized**
**in that** clients (30) which are at least intermittently connected to the server (20) are used to store the data captured by the medical appliance and to inspect, condition, change or add to them using an evaluation application on the clients (30), in that the conditioned, changed or added-to data are transmitted from the clients (30) to the server (20) and are stored in the server (20),
**in that** data are synchronized by transmitting data conditioned, changed or added to exclusively in a client (30) to the server (20),
and **in that** the conditionings, changes or additions performed by further clients (30) and stored on the server (20) are transmitted from the server (20) to the clients (30), so that all clients (30) and the server (20) contain identical data records.

25. Method according to Claim 24, **characterized in that** the data stored in the medical appliance are transmitted from the medical appliance directly to the server (20) or to the client (30).

26. Method according to Claim 24 or 25, **characterized in that** the medical appliance is an external defibrillator (10).

27. The method as claimed in one of the preceding claims, **characterized in that** data are transmitted between the medical appliance and the client (30) by means of an infrared data transmission.

28. The method according to one of the preceding claims, **characterized in that** data are transmitted from the medical appliance to the server (20) or to a communication server (22) connected thereto or from the server (20) or the communication server (22) to the medical appliance using mobile radio technology.

29. Method according to one of the preceding claims, **characterized in that** measured values produced in the course of the treatment are captured and recorded by the medical appliance.

30. Method according to Claims 26 and 29, **characterized in that** data are captured by means of the electrodes (90) of the defibrillator (10) or by means of detectors which receive the data by means of infrared or Bluetooth data transmission.

31. Method according to one of the preceding claims, **characterized in that** a reception unit (122) is provided, and **in that** the data stored in the medical appliance are transmitted to the reception unit (122) by means of infrared or Bluetooth.

32. Method according to Claim 31, **characterized in that** data are transmitted from the server (20) of the reception unit (122) and/or from the reception unit (122) to the server (20).

33. Method according to one of the preceding claims, **characterized in that** the server (20) can be addressed by a fax number, and **in that** the data content of the fax is read into the server (20).

34. Method according to one of the preceding claims, **characterized in that** data stored in the server (20) are sent by fax when requested by the receiver or a third party.

## Revendications

1. Système de communication pour le traitement des données médicales,
le système de communication comprenant appareil médical qui présente des moyens d'acquisition de données médicales, une unité de stockage pour la mémorisation des données appelables ainsi qu'une interface de communication pour la transmission des données,
un serveur (20) qui comprend une unité de stockage dans laquelle des données médicales sont enregistrées de manière appelable, ainsi qu'une interface de communication au moyen de laquelle le serveur (20) est en liaison avec un ou plusieurs clients (30), au moins temporairement,
ainsi que plusieurs clients (30) qui comprennent un unité de traitement ainsi qu'une interface de communication,
**caractérisé en ce que**
les clients (30) comportent en outre une unité de traitement, dans laquelle sont enregistrées les données acquises par l'appareil médical, l'unité de traitement comprenant une application de traitement pour la lecture, la préparation, la modification ou l'adjonction des données enregistrées dans l'unité de traitement des clients (30), les données préparées, modifiées ou complétées dans les clients (30) pouvant être transmises directement des clients (30) au serveur (20) via l'interface de communication, et ces données pouvant être stockées dans l'unité de stockage du serveur (20),
des moyens de synchronisation des données du serveur (20) et des clients (30) sont prévus à l'aide desquels seules les données préparées, modifiées ou complétées dans un client (30) peuvent être transmises au serveur (20) et
des moyens sont prévus à l'aide desquels les préparations, modifications ou adjonctions effectuées par d'autres clients (30) et enregistrées sur le serveur (20) sont transmises du serveur (20) aux clients (30), de sorte que les jeux de données présents dans tous les clients (30) et le serveur (20) sont identiques.

2. Système de communication selon la revendication 1, **caractérisé en ce que** les données enregistrées dans l'appareil médical sont transmissibles directement via son interface de communication, au ou aux clients (30) ou au serveur (20).

3. Système de communication selon la revendication 1 ou 2, **caractérisé en ce que** pour l'appareil médical, il s'agit d'un défibrillateur (10) externe.

4. Système de communication selon l'une des revendications précédentes, **caractérisé en ce que** les préparations, modifications ou adjonctions de données effectuées par d'autres clients et enregistrées sur le serveur (20) peuvent être transmises du serveur (20) au client (30).

5. Système de communication selon l'une des revendications précédentes, **caractérisé en ce que** pour l'application d'utilisation se trouvant sur le client (30), il s'agit d'une utilisation de Java Web Start.

6. Système de communication selon l'une des précédentes revendications, **caractérisé en ce que** l'interface de communication de l'appareil médical est conçue sous la forme d'une interface de téléphonie mobile et **en ce que** le serveur (20) ou un serveur de communication (22) en liaison avec lui dispose aussi d'une interface de téléphonie mobile au moyen de laquelle des données peuvent être transférées de l'appareil médical sur le serveur (20), ou sur le serveur de communication (22) et depuis ce dernier sur le serveur (20).

7. Système de communication selon la revendication 6, **caractérisé en ce que** le serveur de communication (22) est un élément constitutif du serveur (20).

8. Système de communication selon l'une des revendications précédentes, **caractérisé en ce qu'**une interface de communication de l'appareil médical est conçue sous la forme d'une interface de téléphonie mobile et **en ce que** le serveur (20) ou un serveur de communication (22) en liaison avec lui dispose aussi d'une interface de téléphonie mobile au moyen de laquelle des données peuvent être transférées du serveur (20) sur l'appareil médical ou du serveur (20) sur le serveur de communication (22) et depuis ce dernier sur l'appareil médical.

9. Système de communication selon l'une des revendications précédentes, **caractérisé en ce que** sur l'unité de stockage du serveur (20) ou du client (30) peuvent être déposées pour le site de l'appareil médical, des données spécifiques, des mises à jour du firmware, des données relatives aux paramètres matériels de l'appareil médical ou des données du patient, en particulier les données concernant les maladies antérieures et les traitements actuels ou passés et **en ce que** ces données ou mises à jour peuvent être transmises au moyen d'interfaces de communication du serveur (20), du client (30) ou de l'appareil médical, directement depuis le serveur (20) ou le client (30) sur l'appareil médical, ou bien depuis le serveur (20) sur le client (30) et au moyen des interfaces de communication du client (30) et de l'appareil médical, du client (30) sur l'appareil médical.

10. Système de communication selon l'une des précédentes revendications, **caractérisé en ce que** l'appareil médical dispose de moyens d'acquisition de valeurs de mesure produites dans le cadre d'un traitement, les moyens d'acquisition des valeurs de mesure pouvant comprendre des détecteurs, à l'aide desquels les données transmises par infrarouge ou bluetooth, peuvent être acquises par des capteurs (80).

11. Système de communication selon l'une des revendications 3 et 10, **caractérisé en ce que** les moyens d'acquisition des mesures comprennent les électrodes (90) du défibrillateur (10).

12. Système de communication selon l'une des précédentes revendications, **caractérisé en ce que** l'appareil médical comprend des moyens d'acquisition des données du patient.

13. Système de communication selon l'une des précédentes revendications, **caractérisé en ce que** l'appareil médical comprend des moyens (95) pour l'entrée et/ou la sélection de données.

14. Système de communication selon l'une des précédentes revendications, **caractérisé en ce que** l'appareil médical dispose d'une interface de communication au moyen de laquelle les valeurs de mesure produites dans le cadre d'un traitement peuvent être transmises en temps réel sur le moniteur (120).

15. Système de communication selon la revendication 14, **caractérisé en ce que** les valeurs de mesure peuvent être transférées via une interface de communication de l'appareil médical par bluetooth.

16. Système de communication selon l'une des précédentes revendications, **caractérisé en ce que** le système de communication comprend une unité de réception (122) avec une interface de communication pour la réception de données enregistrées dans l'appareil médical de manière appelable, par infrarouge ou bluetooth.

17. Système de communication selon la revendication 16, **caractérisé en ce que** l'unité de réception (122) comprend en outre une interface pour l'échange de données avec le serveur (20).

18. Système de communication selon l'une des précédentes revendications, **caractérisé en ce que** l'appareil médical comporte un moniteur intégré pour la visualisation des données de traitement acquises.

19. Système de communication selon l'une des précédentes revendications, **caractérisé en ce que** le serveur (20) dispose de plusieurs jeux de données, parmi lesquels au moins un premier jeu de données (130) comprend les données du patient et un deuxième jeu de données (140) comporte au moins les données relatives à l'appareil médical, les données enregistrées dans le deuxième jeu de données (140) n'étant pas rattachées à un patient déterminé.

20. Système de communication selon la revendication 19, **caractérisé en ce que** le serveur (20) contient au moins un troisième jeu de données (150) avec les données spécifiques à un médecin et au moins un quatrième jeu de données (160) avec les données spécifiques au site de l'appareil médical.

21. Système de communication selon l'une des précédentes revendications, **caractérisé en ce que** le serveur (20) dispose d'une interface de communication au moyen de laquelle les données des documents transmis par télécopie peuvent être lues dans le serveur (20).

22. Système de communication selon l'une des précédentes revendications, **caractérisé en ce que** le serveur (20) dispose d'une interface de communication au moyen de laquelle des données enregistrées par télécopie peuvent être transmises au serveur (20).

23. Système de communication selon l'une des précédentes revendications, **caractérisé en ce que** des conditions logiques ou des valeurs limites sont enregistrées dans l'unité de stockage du serveur (20) avec les données qui y sont déposées et **en ce que** des moyens sont prévus, à l'aide desquels une alarme ou un message peut être généré lorsque les conditions sont réalisées ou que la valeur limite est atteinte.

24. Procédé de traitement de données médicales dans lequel des données médicales sont saisies dans un appareil médical, enregistrées de manière appelable et transmises à un destinataire et dans lequel les données saisies sur l'appareil médical sont enregistrées de manière appelable dans le serveur (20),
**caractérisé en ce que**
les données saisies sur l'appareil médical sont enregistrées dans les clients (30) connectés au serveur (20), au moins temporairement, et au moyen d'une application de traitement se trouvant sur les clients (30), lues, préparées, modifiées ou complétées, et **en ce que** les données préparées, modifiées ou complétées sont transférées des clients (30) sur le serveur (20) et stockées dans le serveur (20),
**en ce qu'**à des fins de synchronisation des données, seules les données préparées, modifiées ou complétées dans un client (30) sont transmises sur le serveur (20)
et **en ce que** les préparations, modifications ou adjonctions effectuées par d'autres clients (30) et enregistrées sur le serveur (20) sont transmises du serveur (20) aux clients (30), de sorte que des jeux de données identiques se trouvent dans tous les clients (30) et dans le serveur (20).

25. Procédé selon la revendication 24, **caractérisé en ce que** les données enregistrées dans l'appareil médical sont transmises de l'appareil médical directement sur le serveur (20) ou sur le client (30).

26. Procédé selon la revendication 24 ou 25, **caractérisé en ce que** pour l'appareil médical, il s'agit d'un défibrillateur (10) externe.

27. Procédé selon l'une des revendications précédentes, **caractérisé en ce que** la transmission de données entre l'appareil médical et le client (30) s'effectue par une transmission de données infrarouge.

28. Procédé selon l'une des revendications précédentes, **caractérisé en ce que** les données sont transmises par téléphonie mobile de l'appareil médical au serveur (20) ou à un serveur de communication (22) connecté à celui-ci, ou du serveur (20) ou encore du serveur de communication (22) à l'appareil médical.

29. Procédé selon l'une des revendications précédentes, **caractérisé en ce que** les valeurs de mesure produites dans le cadre du traitement sont saisies et enregistrées par l'appareil médical.

30. Procédé selon la revendication 26 et 29, **caractérisé en ce que** l'acquisition des données s'effectue au moyen des électrodes (90) du défibrillateur (10) ou au moyen de détecteurs qui reçoivent les données via une transmission de données par infrarouge ou bluetooth.

31. Procédé selon l'une des revendications précédentes, **caractérisé en ce qu'**une unité de réception (122) est prévue et **en ce que** les données enregistrées dans l'appareil médical sont transmises à l'unité de réception (122) par infrarouge ou bluetooth.

32. Procédé selon la revendication 31, **caractérisé en ce que** les données sont transmises du serveur (20) à l'unité de réception (122) et/ou de l'unité de réception (122) au serveur (20).

33. Procédé selon l'une des revendications précédentes, **caractérisé en ce que** le serveur (20) est adressable par un numéro de télécopieur et **en ce que** le contenu des données de la télécopie est lu dans le serveur (20).

34. Procédé selon l'une des revendications précédentes, **caractérisé en ce que** les données enregistrées dans le serveur (20) sont envoyées par télécopie sur demande du destinataire ou d'un tiers.
